# EUROPÄISCHE PATENTANMELDUNG

(11) **EP 1 842 843 A1**
(43) Veröffentlichungstag der Anmeldung: **10.10.2007**
(21) Anmeldenummer: 06007118.0
(22) Anmeldetag: 04.04.2006
(51) Int. Cl.: C07C 67/08, C07C 69/40, C07D 307/08, C07D 307/32, C07C 29/149, C07C 31/20

(54) **Verfahren zur Herstellung eines Carbonsäurealkylesters**

(71) Anmelder: BASF Aktiengesellschaft, 67056 Ludwigshafen (DE)
(72) Erfinder: Bauduin,Christophe, 68259 Mannheim (DE); Fischer,Wolfgang, 67360 Lingenfeld (DE); Pinkos, Rolf., 67098 Bad Dürkheim (DE); Scholten, Edzard, 68159 Mannheim (DE)
(74) Vertreter: Reitstötter - Kinzebach

(57) **Zusammenfassung**

Die vorliegende Erfindung betrifft ein neuartiges Verfahren zur Herstellung von Polycarbonsäurealkylestern aus einer wässrigen Lösung eines Ammoniumsalzes der Polycarbonsäure durch Reaktivdestillation, sowie ein Verfahren zur Hydrierung der auf diese Weise hergestellten Carbonsäurealkylester.

## Beschreibung

Die vorliegende Erfindung betrifft ein neuartiges Verfahren zur Herstellung von Polycarbonsäurealkylestern aus einer wässrigen Lösung eines Ammoniumsalzes der Polycarbonsäure sowie ein Verfahren zur Hydrierung der auf diese Weise hergestellten Carbonsäurealkylester.

### Hintergrund der Erfindung

Organische Carbonsäuren und deren Ester stellen wichtige Ausgangsprodukte für die chemische Synthese dar. Von besonderer wirtschaftlicher Bedeutung ist es dabei, derartige organische Carbonsäuren und deren Ester möglichst kostengünstig herzustellen. Die fermentative Herstellung von Carbonsäuren unter Verwendung geeigneter Mikroorganismen gewinnt zunehmende Bedeutung. Nachteilig bei diesen Verfahren ist jedoch, dass die Carbonsäuren meistens nicht als Reinsubstanz und zudem nicht in Form der freien Säure, sondern teilweise oder vollständig neutralisiert in Salzform anfallen. Eine Neutralisierung ist meistens notwendig, da anderenfalls bei zunehmender Fermentationsdauer die Fermentationsbrühe übersäuern und als folge davon der Mikroorganismus geschädigt würde. Die Reindarstellung fermentativ aber auch chemisch synthetisierter Carbonsäuren bzw. der korrespondierenden Ester ist daher von großer Bedeutung.

Ausgehend von fermentativ hergestellten Carbonsäuren, wie insbesondere Milchsäure oder Bernsteinsäure, sind mit Hilfe verschiedener Synthesestrategien unterschiedlichste Wertprodukte auf chemischem Weg zugänglich (vgl. z.B. Übersichtsartikel von Varadarajan et al., Biotechnol. Proc. 1999, 15, 845-854).

Im Stand der Technik werden außerdem verschiedene Strategien zur Aufarbeitung von Carbonsäuren, insbesondere zur Veresterung von Carbonsäuren beschrieben. So befasst sich beispielsweise die WO-A-00/64850 mit der Herstellung von organischen Säuren aus den korrespondierenden Ammoniumsalzen durch thermische Zersetzung einer wässrigen Ammoniumsalzlösung der organischen Säure in Gegenwart eines Alkohols und Entfernung eines dampfförmigen Gemisches aus Ammoniak, Wasser und Alkohol aus dem Reaktionsgemisch. Falls gewünscht, kann die gebildete organische Säure in einen Ester überführt werden. Offensichtlich ist jedoch bei diesem Verfahren die maximale Esterausbeute sehr gering, so wird in den dortigen Ausführungsbeispielen für Ethyllactat eine maximale Ausbeute von nur 14,6 % beschrieben. Für die quantitative Überführung eines Ammoniiumsalzes einer organischen Säure in den korrespondierenden Ester scheint dieses Verfahren somit nicht geeignet zu sein. Darüber hinaus ist die Brauchbarkeit des dort beschriebenen Systems zur Veresterung von Polycarbonsäuren durch keinerlei Beispiele belegt.

Die Veresterung von fermentativ hergestellten Carbonsäuresalzen durch katalytische Umsetzung eines Salz/Alkoholgemisches und anschließende Entfernung des gebildeten Ammoniaks und Wassers durch Pervaporation wird in der WO-A-98/23579 beschrieben. Nachteilig bei diesem Verfahren ist jedoch die Notwendigkeit der Verwendung von Pervaporationsmembranen. Darüber hinaus werden in den dortigen Ausführungsbeispielen Veresterungsraten von maximal 40,7 % (bei der Umsetzung von Ammoniumpropionat zum korrespondierenden Ethylester) beschrieben. Somit scheint auch dieses Verfahren für eine möglichst quantitative Veresterung von Ammoniumsalzen von Carbonsäuren nicht geeignet zu sein. Darüber hinaus ist die Brauchbarkeit des dort beschriebenen Systems zur Veresterung von Polycarbonsäuren durch keinerlei Beispiele belegt.

Die ein- oder mehrstufige Veresterung freier Mono-, Di- oder Polycarbonsäuren durch Umsetzung eines Carbonsäure/Alkoholgemisches in einem Reaktor ist in der WO-A-2005/051885 beschrieben. Es findet sich aber kein Hinweis die Brauchbarkeit dieses Verfahrens für die Veresterung entsprechender Ammoniumsalze.

Die Bereitstellung esterhaltiger Brennstoffe und Chemikalien aus Biomasse durch Umsetzung biologisch hergestellter Alkohole, insbesondere Methanol und Ethanol, und fermentativ hergestellter Carbonsäuren, insbesondere deren Ammoniumsalze, wird von Olson et al. in Applied Biochemistry and Biotechnology, 2003, Vol. 105-108, 843-851, vorgeschlagen. Verschiedene Methoden zur Herstellung von Monocarbonsäureestern werden diskutiert. Die erfolgreiche Veresterung von Polycarbonsäuresalzen wird in diesem Dokument nicht beschrieben.

Die DE-A-198 29 809 beschreibt die Veresterung eines Gemischs aus Wasser und freier Monocarbonsäure durch Reaktivdestillation.

Keines der zitierten Dokumente befasst sich zudem mit der gezielten Vermeidung von unerwünschten stickstoffhaltige Nebenprodukten der Veresterungsreaktion, insbesondere von chemisch stabilen Carbonsäureimiden, deren Bildung bei der Veresterung von Carbonsäureammoniumsalzen ein signifikantes Problem darstellt. Derartige Imide sind zudem aus dem Reaktionsgemisch nur schwer zu entfernen und können bei späteren chemischen Umsetzungen störend wirken.

Der vorliegenden Erfindung liegt daher die Aufgabe zugrunde, ein verbessertes Verfahren zur Herstellung von Polycarbonsäureestern aus den korrespondierenden Ammoniumsalzen bereitzustellen, welche einerseits eine hohe Esterausbeute gewährleisten und andererseits die Bildung unerwünschter stickstoffhaltiger Nebenprodukte, wie insbesondere von Carbonsäureimiden, weitgehend verhindern.

### Figurenbeschreibung

Figur 1 zeigt in schematischer Darstellung ein Beispiel einer Vorrichtung zur Durchführung der erfindungsgemäßen Veresterung durch Reaktivdestillation in einer Glockenbodenkolonne, welche in semi-batch-Fahrweise betrieben wird.

Figur 2 zeigt in schematischer Darstellung ein weiteres Beispiel einer Vorrichtung zur Durchführung der erfindungsgemäßen Veresterung durch Reaktivdestillation in einer Kolonne mit Einbauten, hier z.B. einer Glockenbodenkolonne, welche in kontinuierlicher Fahrweise betrieben wird.

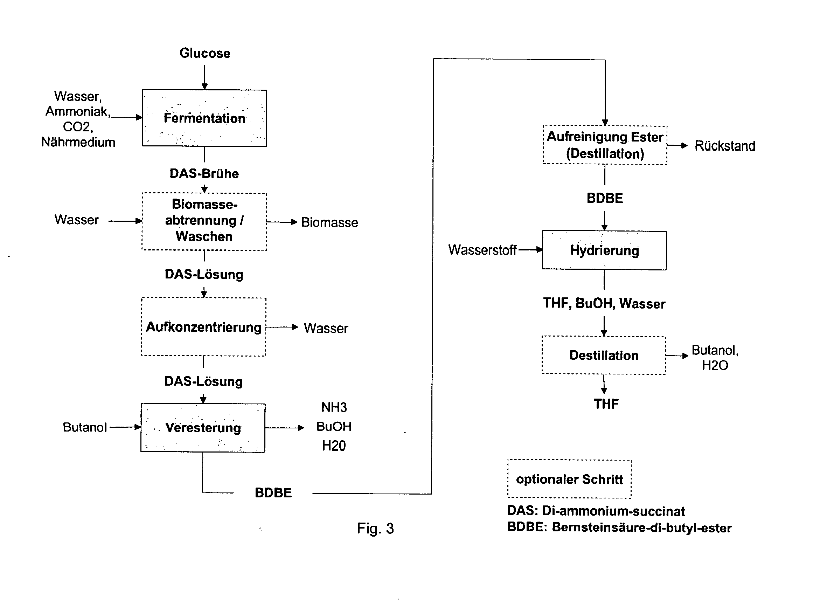

Figur 3 zeigt das Fließdiagramm eines erfindungsgemäßen Gesamtprozesses zur Herstellung von Tetrahydrofuran, ausgehend von einer Fermentation mit Glucose als Substrat über die Veresterung des dabei anfallenden Diammoniumsalzes von Bernsteinsäure (DAS) mit Butanol zu Bernsteinsäure-di-butylester (BDBE) und die katalytische Hydrierung des Esters zu Tetrahydrofuran (THF). Optionale Verfahrensschritte sind durch gestrichelte Rahmen hervorgehoben. Mögliche Abwandlungen bzw. Ergänzungen dieses Gesamtprozesses, wie z.B. die Rückführung von freigesetztem Butanol und/oder die Rückführung von freigesetztem Ammoniak, sind lediglich aus Gründen der Übersichtlichkeit nicht angegeben.

### Kurzfassung der Erfindung

Obige Aufgabe konnte überraschenderweise durch Bereitstellung eines Verfahrens zur Herstellung eines Polycarbonsäureesters gelöst werden, bei welchem man eine Reaktivdestillation durchführt, indem man eine Vorlage, umfassend eine wässrige Lösung eines Ammoniumsalzes der Polycarbonsäure in einer Destillationskolonne mit einem Alkanol in Kontakt bringt und bei der Veresterung freigesetztes Ammoniak sowie Wasser destillativ aus der Reaktion entfernt.

Bei Befolgung der erfindungsgemäßen Lehre gelingt es überraschenderweise den gewünschten Polycarbonsäureester in deutlich verbesserter Ausbeute und überraschend hoher Reinheit bereitzustellen. So können Veresterungsausbeuten von 80 bis 100%, wie z.B. 90 bis 99 oder 92 bis 98 % bezogen auf die eingesetzte Carbonsäuremenge erzielt werden.

Insbesondere kann der Gehalt and unerwünschten Stickstoffverbindungen im Produkt signifikant verringert werden, so dass eine direkte katalytische Hydrierung des Esters ermöglicht wird.

### Detaillierte Beschreibung der Erfindung

### 1. Bevorzugte Ausführungsformen der Erfindung:

Ein erster Gegenstand der Erfindung betrifft ein Verfahren zur Herstellung eines Carbonsäurealkylesters, insbesondere Polycarbonsäureesters, wobei man eine Reaktivdestillation durchführt, indem man eine Vorlage, umfassend eine wässrige Lösung eines Ammoniumsalzes der Carbonsäure in wenigstens einer Destillationskolonne, insbesondere einer oder zwei miteinender in an sich bekannter Weise verbundenen Destillationskolonnen, mit Alkanol beispielsweise im Gleichstrom oder Gegenstrom in Kontakt bringt und bei der Veresterung anfallendes Ammoniak und Wasser destillativ aus der Reaktion entfernt, und wobei sich der gebildete Carbonsäureester bzw. dessen Vorprodukte (wie z.B. im Falle von Bernsteinsäuredibutylester der Monoester und Bernsteinsäureanhydrid) beispielsweise im Sumpf der Kolonne anreichert, beziehungsweise von dort aus der Kolonne ausgeleitet wird. Falls erforderlich können an einer oder mehreren und/oder zwischen mehreren Destillationskolonnen ein oder mehrere Verweilzeitbehälter in an sich bekannter Weise vorgesehen sein, um Umsatz zu verbessern, z.B. um Vollumsatz zu erreichen.

Der Ammoniumpolycarbonsäuresalz-Gehalt der Vorlage liegt gewöhnlich im Bereich von etwa 1 bis 60 oder 10 bis 50 oder 30 bis 45 Gew.-%, bezogen auf das Gesamtgewicht der Vorlage. Der Wassergehalt sollte natürlich so gering wie möglich sein, andererseits sollte er aber hoch genug sein, um die Carbonsäuresalze in Lösung zu halten.

Das erfindungsgemäße Verfahren kann dabei in unterschiedlicher Fahrweise, wie in Batch-Fahrweise, Semi-Batch-Fahrweise oder kontinuierlich, durchgeführt werden.

Insbesondere bei kontinuierlicher Fahrweise ist es von Vorteil, wiederverwendbare Nebenprodukte der Umsetzung, wie z.B. Ammoniak, oder nicht-umgesetzten bzw. in späterem Reaktionsschritt frei werdender Alkohol, an geeigneter Stelle in das Verfahren wieder zurückzuführen. Ammoniak kann beispielsweise zur Neutralisierung der Polycarbonsäure wiederverwendet werden.

Das erfindungsgemäß eingesetzte Salz einer Carbonsäure ist vorzugsweise ausgewählt unter jeweils gesättigten oder ungesättigten, aliphatischen C₃ - C₂₀-Polycarbonsäuren, d.h. Carbonsäuren mit zwei oder mehr, wie z.B. 2, 3 oder Carboxylgruppen, wie insbesondere Dicarbonsäuren, vorzugsweise gesättigten Dicarbonsäuren, und Mischungen davon. Dabei ist die Polycarbonsäure mit Ammoniak teilweise oder vollständig neutralisiert. Besonders bevorzugt ist das eingesetzte Carbonsäure-Ammoniumsalz Diammoniumsuccinat.

Insbesondere wird weiterhin ein Alkanol für die Veresterung eingesetzt, das ausgewählt ist unter geradkettigen oder verzweigten C₁ - C₆- Monoalkanolen. Nach einer bevorzugten Variante ist das eingesetzte Alkanol n-Butanol und man erhält als Veresterungsprodukt Di-n-butyl-succinat erhält.

In einer weiteren Ausgestaltung der Erfindung wird die Reaktion in Gegenwart eines Veresterungskatalysators durchgeführt.

Weiterhin ist bevorzugt, die wässrige Carbonsäuresalz-haltige Lösung in der oberen Hälfte der Destillationskolonne, beispielsweise in Höhe des Kondensatorrücklaufs, zuzuführen.

Die bei der Reaktion freigesetzten Ammoniak und Wasser kann man über den Kolonnenkopf abdestillieren, wobei man ein Alkanol/Wasser-Gemisch oder-Azeotrop (je nach verwendetem Alkanol) und Ammoniak abdestilliert, kondensiert, aus dem Kondensat das Alkanol gewinnt und in die Umsetzung zurückführt, oder ein Alkanol/Wasser-Hetero-Azeotrop und Ammoniak abdestilliert, kondensiert, aus dem Kondensat die organische Alkanol-Phase abtrennt (wie z.B. durch Phasentrennung oder durch Destillation) und in die Umsetzung zurückführt. Azeotrope Gemische können insbesondere bei Verwendung von Ethanol, Propanol und Butanol gebildet werden ; Hetreo-Azeotrope bei Verwendung von Pentanol und höheren Alkoholen.

Fallen bei der Reaktivdestillation leichterflüchtige Komponenten als das abzutrennende Alkohol-haltige Azeotrop oder Alkohol-Wassergemisch an, so kann diese leichterflüchtige Komponente zusammen mit Ammoniak über Kopf abgezogen und das Alkohol-haltige Gemisch bzw. Azeotrop unterhalb des Kolonnenkopfes seitlich abgezogen werden.

Eine bevorzugte Verfahrensvariante umfasst die Verwendung eines homogenen oder eines heterogenen Veresterungskatalysators, wobei der heterogene Katalysator insbesondere ein saurer lonenaustauscher-Katalysator ist und der heterogene Katalysator auf oder in Kolonneneinbauten fixiert ist.

In einer bevorzugte Ausführungsform verwendet man einen homogenen Katalysator, wie z.B. p-Toluolsulfonsäure, den man der wässrigen Ammoniumsalz-Lösung der Carbonsäure zusetzt.

Das im erfindungsgemäßen Veresterungsverfahren eingesetzte Carbonsäure-Ammoniumsalz kann entweder chemisch hergestellt sein oder eine gegebenenfalls vorgereinigte und/oder aufkonzentrierte Fermentationsbrühe sein, die ein oder mehrere Carbonsäure-Ammoniumsalze enthält. Die Fermentationsbrühe kann auch freie Säure oder teilweise neutralisierte Salze enthalten und zur Umsetzung können diese durch Zugabe von Ammoniumionen, wie z. B. durch Zugabe von Ammoniumhydroxid oder Ammoniumcarbonat, teilweise oder vollständig neutralisiert werden. Geeignete Fermentationsbrühen stammen beispielsweise aus der Kultivierung eines Pentosen und/oder Hexosen, insbesondere Glucose-fermentierenden Mikroorganismus.

Ein weiterer Gegenstand der Erfindung betrifft ein Verfahren zur Hydrierung von Polycarbonsäurealkylestern, wobei man zunächst eine Veresterungsreaktion nach obiger Definition durchführt, aus dem dabei anfallenden Ester-haltigen Reaktionsgemisch den Polycarbonsäurealkylester gegebenenfalls aufreinigt, wie z.B. mittels Destillation, den Ester katalytisch hydriert und das gewünschte Wertprodukt isoliert. Dabei verwendet man für die Hydrierung vorzugsweise einen geträgerten CuO-Katalysator.

Besonders bevorzugt wird dabei Di-n-butyl-succinat zu Tetrahydrofuran, 1,4-Butandiol gamma-Butyrolacton oder Mischungen dieser Verbindungen hydriert.

### 2. Weitere Ausgestaltungen der Erfindung

### a) Reaktivdestillation des Esters

Unter einer "Reaktivdestillation" versteht man erfindungsgemäß die simultane Durchführung einer chemischen Reaktion und einer Destillation bzw. Rektifikation. Diese simultane Durchführung von Reaktion und Stofftrennung ist besonders vorteilhaft bei solchen Reaktionen, bei denen aufgrund der Lage des chemischen Gleichgewichtes die Edukte nicht vollständig in die gewünschten Produkte umgesetzt werden. Durch die simultane Abtrennung der Reaktionsprodukte aus dem Reaktionsraum gelingt dabei eine fast vollständige Umsetzung in einem einzigen Vorrichtung. Weitere Vorteile der Reaktivdestillation sind in der Unterdrückung unerwünschter Nebenreaktionen, der thermischen Nutzung einer exothermen Reaktionswärme für die Destillation und in der Erleichterung der nachfolgenden Produktaufbereitung zu sehen.

Die apparative Durchführung der erfindungsgemäßen Reaktivdestillation ist in verschiedenen Ausgestaltungen möglich. Im Prinzip eignen sich hierzu alle bekannten Bauformen von Destillations- bzw. Rektifikationskolonnen brauchbar.

Eine "Rektifikationskolonne" besteht gewöhnlich aus einem Verdampfer am Boden, einem Kondensator am Kopf und verschiedenen Einbauten in der Kolonne. Je nach Art der verwendeten Einbauten wird unterschieden zwischen Boden-, Füllkörper- und Packungskolonnen. Durch einen Zulauf wird die zu trennende Substanz eingespeist. Im Kopf reichern sich die leichter siedenden Komponenten an und können dort abgetrennt werden. Um die Trennung in der Kolonne zu erreichen, wird gewöhnlich ein Teilstrom des Kondenats wieder in die Kolonne zurückgeleitet. Im Sumpf reichern sich die schwerer siedenden Komponenten an und können dort abgenommen werden. Es besteht zudem die Möglichkeit, je nach Trennproblem, durch einen Seitenabzug Komponenten zu gewinnen, die zwischen den am leichtesten und den am schwersten siedenden Komponenten liegen.

In typischen Bodenkolonnen sind Sieb-, Glocken- oder Ventilböden eingebaut, über welche die Flüssigkeit strömt. Durch spezielle Schlitze oder Löcher wird der Dampf geleitet, so dass eine Sprudelschicht entsteht. Auf jedem dieser Böden stellt sich ein neues Verdampfungsgleichgewicht ein.

Füllkörperkolonnen können mit unterschiedlich geformten Füllkörpern gefüllt werden. Durch die damit verbundene Oberflächenvergrößerung der etwa 3-50 mm großen Füllkörper werden Wärme- und Stoffaustausch optimiert und die Trennfähigkeit der Kolonne somit erhöht. Typische Beispiele für solche Substrate sind der Raschig-Ring (ein Hohlzylinder), Pall-Ring, Hiflow-Ring, Intalox-Sattel, Berl-Sattel und Igel. Die Füllkörper können geordnet, aber auch regellos (als Schüttung) in die Kolonne eingebracht werden. Als Materialien kommen in Frage Glas, Keramik, Metall und Kunststoffe.

Strukturierte Packungen sind eine Weiterentwicklung der geordneten Füllkörper. Sie weisen eine regelmäßig geformte Struktur auf. Es gibt verschiedene Ausführungen von Packungen z. B. Gewebe- oder Blechpackungen. Als Material können Metall, Kunststoff, Glas und Keramik eingesetzt werden. Packungskolonnen haben im Vergleich zu Bodenkolonnen einen sehr geringen Flüssigkeitsinhalt. Dies ist für die Rektifikation oft von Vorteil, da dadurch die Gefahr der thermischen Zersetzung der Substanzen vermindert wird.

Für die Reaktivdestillation ist der geringe Flüssigkeitsinhalt von Packungskolonnen nicht immer zweckmäßig, insbesondere bei Reaktionen mit endlicher Reaktionsgeschwindigkeit. Um den gewünschten Umsatz bei langsamen Reaktionen zu erzielen, sollte eine hohe Verweilzeit der Flüssigkeit im Apparat gewährleistet werden. Zu diesem Zweck kann man sich oft dadurch behelfen, dass neben oder nach der Kolonne, insbesondere neben der Packungskolonne, ein oder mehrere externe Behälter geschaltet werden, in denen ein Großteil der eigentlichen Reaktion stattfindet.

Für die erfindungsgemäße Durchführung der Reaktivdestillation ist der Einsatz von Bodenkolonnen eine besonders geeignete Variante. Bodenkolonnen haben einen hohen Flüssigkeitsinhalt, und zwar sowohl in der Zweiphasenschicht auf dem Boden als auch in den Ablaufschächten. Beide Anteile des Flüssigkeitsinhaltes können durch konstruktive Maßnahmen, d.h. durch die Gestaltung der Böden, gezielt verändert und an die jeweiligen Anforderungen der Reaktivdestillation angepasst werden. Der Entwurf von optimalen Einbauten für eine erfindungsgemäß optimierte reaktive Bodenkolonne liegt im Bereich des fachmännischen Könnens. Insbesondere kann der Fachmann geeignete konstruktive Maßnahmen zur Realisierung geeigneter Flüssigkeitsinhalte, und somit geeigneter Verweilzeiten sowie zur gegebenenfalls erforderlichen Befüllung mit heterogenem Katalysator treffen.

Ein großer Flüssigkeitsinhalt in der Zweiphasenschicht auf den Böden lässt sich am einfachsten durch die Verwendung hoher Überlaufwehre einstellen. Nachteilig kann hierbei ein hoher Druckverlust des Dampfes und die große erforderliche Bauhöhe der Kolonne sein. Vorteilhafter erscheint die Realisierung eines großen Flüssigkeitsinhaltes in den Ablaufschächten da hierbei kein zusätzlicher Druckverlust entsteht. In diesem Fall können Reaktion und Destillation an jedem Boden weitgehend getrennt ablaufen und zwar die Reaktion im Ablaufschacht und die Destillation in der Zweiphasenschicht auf dem Boden. Eine weitere denkbare Modifikation wäre die Verwendung einer abwechselnden Folge von Austauschböden und Kamin- bzw. Reaktionsböden mit hohem Flüssigkeitsinhalt

### b) Geeignete Reaktanden und Veresterungskatalysatoren

Erfindungsgemäß veresterbare Polycarbonsäuren weisen 2 oder mehr, insbesondere 2, 3 oder 4, vorzugsweise 2 Carboxylgruppen auf. Insbesondere sind dies geradkettige oder verzweigte aliphatische, oder cycloaliphatische, gesättigte oder ungesättigte Polycarbonsäuren mit 2 bis 20, wie z.B. 2 bis 10 oder 3 bis 8, insbesondere 4, 5 oder 6 Kohlenstoffatomen, welche gegebenenfalls mit einer oder mehreren Hydroxylgruppen substituiert sind. Als konkrete Beispiele sind zu nennen Dicarbonsäuren, wie Oxalsäure, Maleinsäure, Itaconsäure, Fumarsäure, Bernsteinsäure, Methylbernsteinsäure, Glutarsäure, 2- oder 3-Methylglutarsäure, Adipinsäure sowie deren hydroxysubstituierten Derivate, wie z.B. Äpfelsäure oder 2-Hydroxy-Glutarsäure, oder Gemische davon.

Als Beispiele für höherwertige Polycarbonsäuren sind zu nennen Citronensäure, Agaricinsäure, Propan-1,2,3-tricarbonsäure (Tricarballylsäure), Aconitsäure oder Gemische davon.

Als erfindungsgemäß brauchbare Alkanole sind Monoole zu nennen. Besonders brauchbar sind C₁-C₁₂-, C₁-C₁₀-, C₁-C₈- oder C₃-C₆-Alkanole. Als Beispiele sind zu nennen Monoole deren Alkylteil ausgewählt ist unter Methyl, Ethyl, n-Propyl, Isopropyl, n-Butyl, sec-Butyl, Isobutyl, tert-Butyl, Pentyl, Hexyl, Heptyl, Octyl, 2-Ethylhexyl, Nonyl, Decyl, Undecyl oder Dodecyl sowie die dazugehörigen Stellungsisomere.

Das Molare Verhältnis von Carbonsäure bzw. -salze zu Alkanol liegt gewöhnlich im Bereich von 1:1 bis 1:10, wie z.B. 1:1 bis 1:5 oder 1:1 bis 1:2.

Beispiele für geeignete feste Veresterungskatalysatoren sind Ionenaustauscherharze, erhältlich unter den Handelsbezeichnungen Amberlyst 15 oder Amberlyst 16 (Rohm und Haas) oder DPT1 (Davy Process Technology Limited).

Geeignete flüssige Veresterungskatalysatoren sind Schwefelsäure, p-Toluolsulfonsäure, 4-Dimethylaminopyridine (DMAP), oder Mischungen von tertiären C₈ - C₁₀-Aminen erhältlich unter der Handelsbezeichnung Alamine 336 (Fa. Henkel)

### c) Veresterungsverfahren

Das erfindungsgemäße Veresterungsverfahren, welches im Wesentlichen eine Reaktivdestillation umfasst, ist unter Verwendung an sich bekannter technischer Vorrichtungen in verschiedenen Ausgestaltungen durchführbar. Im Folgenden werden zwei nicht limitierende, spezielle Beispiele erfindungsgemäßer Verfahrensweisen anhand der beiliegenden Figuren näher erläutert.

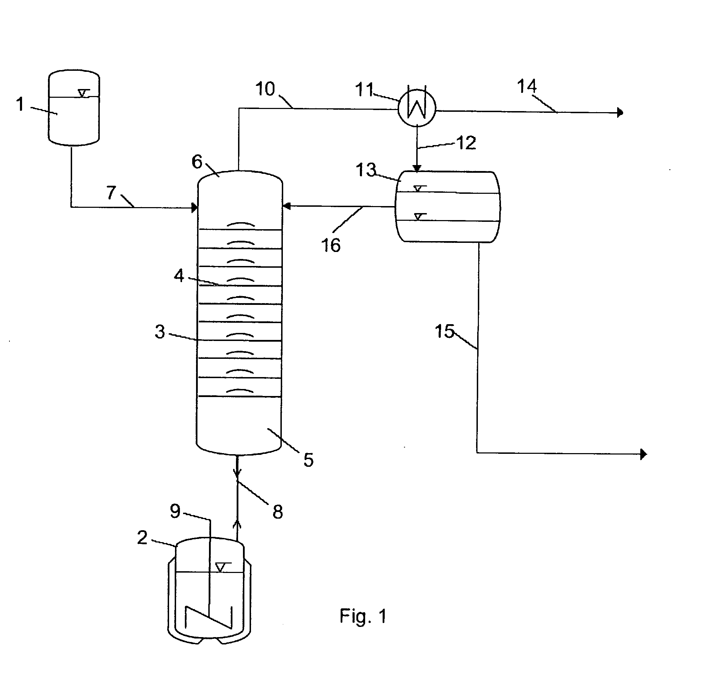

Figur 1 veranschaulicht ein Beispiel für eine in semi-batch-Fahrweise betriebene Veresterungsanlage welche eine Glockenbodenkolonne 3 mit einer Vielzahl von Glockenböden 4 umfasst. In den Kolonnenkopf 6 wird aus dem Vorlagenbehälter 1 über die Zuleitung 7 die wässrige, das Ammoniumsalz der Polycarbonsäure enthaltende Vorlage eingespeist, sobald es sich in der Kolonne 3 durch Einspeisung von dampfförmigem Alkanol ein stationäres Temperaturprofil ausgebildet hat. Die Alkoholvorlage befindet sich dabei in dem beheizten, mit einem Rührer 9 versehenen Rührbehälter 2, der über die Verbindungsleitung 8 mit dem unteren Ende 5 der Kolonne 3 verbunden ist. Bei der Reaktion bildet sich ein Gegenstrom von absteigender, ammoniumsalzhaltigem Flüssigkeit und Kondensat und aufsteigender, alkanolhaltiger Dampfphase aus. Zur Katalyse der Veresterungsreaktion kann der Ammoniumsalzvorlage 1 ein homogener Katalysator zugesetzt werden. Alternativ dazu können heterogene Katalysatoren in den Kolonneneinbauten, wie z.B. in den Böden 4, vorgesehen sein. Der gebildete Carbonsäureester ist unter den Verfahrensbedingungen flüssig und gelangt über das untere Ende der Kolonne 5 und die Verbindungsleitung 8 in den Rührbehälter 2, welcher somit gleichzeitig den Sumpf der Destillationskolonne 3 bildet. Über die am Kolonnenkopf 6 angebrachte Brüdenleitung 10 werden gasförmige Komponenten, wie z.B. azeotrope Mischungen umfassend Alkanol-Wasser und/oder Ammoniak aus der Reaktionskolonne 3 und damit aus dem Reaktionsgleichgewicht entfernt. Durch Passieren des Kondensators 11 werden verflüssigbare Anteile der ausgeleiteten Phase verflüssigt und über die Ableitung 12 in das Phasentrenngefäß 13 geführt. Nicht verflüssigbare Anteile, wie insbesondere Ammoniak, werden über die Abgasleitung 14 abgeleitet. Im Phasentrenngefäß 13 erfolgt eine Trennung des Kondensats in organische und wässrige Phase. Die im Wesentlichen Wasser enthaltende wässrige Phase wird über die Ableitung 15 ausgeleitet. Über die Leitung 16 wird die Alkanol-haltige organische Phase in den Kolonnenkopf 6 und somit in den Prozess zurückgeführt.

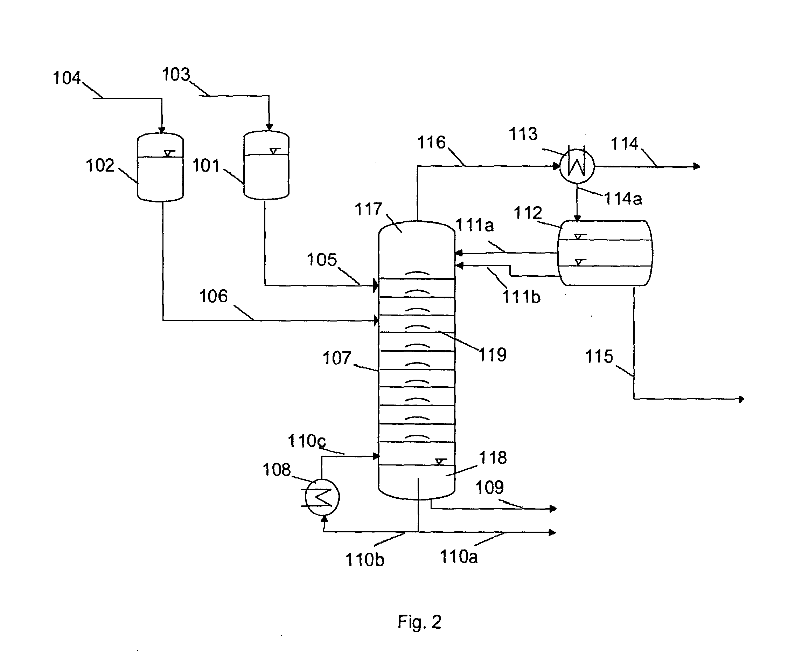

Figur 2 zeigt ein Beispiel einer kontinuierlich betriebenen Destillationsanlage, welche wiederum eine Glockenbodenkolonne 107 mit einer Vielzahl von Glockenböden 119 umfasst. Die Vorrichtung umfasst weiterhin Vorlagenbehälter 101 und 102 für die wässrige Polycarbonsäure- Ammoniumsalzlösung bzw. für den Alkohol. Über die Zuleitung 103 wird Ammoniumsalzlösung, gegebenenfalls im Gemisch mit homogenem Katalysator, aus einer Vorstufe, beispielsweise der fermentativen Herstellung einer Ammoniumsalz-haltigen Fermentationsbrühe kontinuierlich eingespeist. Über die Zuleitung 104 wird reiner Alkohol bzw. Alkohol aus der Rückführung des kontinuierlichen Prozesses in den Vorlagenbehälter 102 kontinuierlich zugeführt. In die Reaktivkolonne 107 wird über die Leitung 106 Butanol eingespeist, welches über den Sumpf 118 der Kolonne und die Rückführung 110b in den Verdampfer 108 gelangt und als Dampf über die Zuleitung 110 in die Kolonne gelangt. Sobald sich ein stationäres Temperaturprofil ausgebildet hat, wird aus dem Vorlagenbehälter 101 über die Zuleitung 105 Ammoniumsalzlösung im oberen Teil (Kolonnenkopf) 117 der Kolonne eingespeist. Es bildet sich ein Gegenstrom von nichtveresterte Carbonsäure enthaltender Flüssigkeit und Kondensat und aufsteigender alkoholhaltiger Dampfphase aus. Bei den Verfahrensbedingungen flüssige, während der Reaktion gebildete Polycarbonsäureester bzw. Vorstufen, sammeln sich im Sumpf 118 der Kolonne 107 an. Gasförmige Komponenten, wie insbesondere azeotrope Mischungen umfassend Alkohol, Wasser und/oder Ammoniak, werden über den Kolonnenkopf 117 und die Ableitung 116 aus der Kolonne 107 in den Kondensator 113 geleitet und dort in gasförmige und flüssige Komponenten getrennt. Nicht verflüssigbare Bestandteile werden über die Abgasleitung 114 ausgeleitet. Flüssige Bestandteile gelangen über die Ableitung 114a in das Phasentrenngefäß 112. Nach Phasentrennung wird über die Rückführung 111 a die organische Phase in den Kolonnenkopf 117 zurückgeführt und die wässrige Phase über die Abeitung 115 abgezogen. Optional kann insbesondere ein Teil der wässrigen Phase auch über eine Leitung 111 b in die Kolonne 107 zurückgeführt werden. Die sich im Sumpf 118 der Kolonne ansammelnde esterhaltige Flüssigphase wird über die Leitung 110a aus der Kolonne kontinuierlich abgezogen werden. Optional kann auch über einen Anzug 109 im Boden des Sumpfes 118 gebildeter oder angesammelter Rückstand abgezogen werden. Anstelle eines homogenen Katalysators, kann in der Kolonne 107 auch ein heterogener Katalysator verwendet werden, der im Bereich der Kolonneneinbauten 119 vorgesehen ist. Eine Rückführung von aufgearbeiteten Alkohol, z. B. aus der Wasserphase, über die Leitung 104 in die Alkohol-Vorlage 102 ist möglich.

Weitere Abwandlungen der oben beschriebenen konkreten Ausführungsformen erfindungsgemäß brauchbarer Vorrichtungen sind im Prinzip denkbar und vom Fachmann ohne unzumutbaren Aufwand verwirklichbar.

Geeignete Verfahrensparameterbereiche für das erfindungsgemäße Veresterungsverfahren sind vom Fachmann in Abhängigkeit von der Ausgestaltung der verwendeten Vorrichtung, wie z.B. Art der verwendeten Kolonneneinbauten, Art und Menge der Reaktanden, Art und Menge des gegebenenfalls verwendeten Katalysators leicht bestimmbar. So können, ohne darauf beschränkt zu sein, einzelne Parameter innerhalb folgender Parameterbereiche eingestellt werden:
Kolonnentemperatur: 0 - 300 °C, insbesondere 40 - 250 °C, oder 70 - 200 °C
Druck: 0,1 bar bis 6, insbesondere Normaldruck.
Verweilzeit: wenige Sekunden (wie z.B. 1 bis 60) bis zu Tagen (wie z.B. 1 bis 5), insbesondere einige Minuten(wie z.B. 1 bis 60) bis einige Stunden (wie z.B. 1 bis 15), besonders bevorzugt einige Minuten (wie z.B. 5 bis 20) bis 2 h

### d) Hydrierverfahren

Die Hydrierung der erfindungsgemäß hergestellten Polycarbonsäureester erfolgt in an sich bekannter Weise unter Anwendung dem Fachmann geläufiger Verfahren, Vorrichtungen und Hilfsmittel, wie Katalysatoren.

Insbesondere wird eine kontinuierliche oder diskontinuierliche Gasphasenhydrierung in Gegenwart eines heterogenen, für die Esterhydrierung geeigneten Katalysators durchgeführt. Die optimalen Verfahrensparameter können vom Fachmann für den jeweiligen Ester ohne unzumutbaren Aufwand eingestellt werden. So liegt beispielsweise die Reaktionstemperatur im Bereich von etwas 100 bis etwa 300 °C, vorzugsweise im Bereich von etwa 200 bis 280 °C und der Druck liegt bei etwa 5 bis 100 bar, wie z. B. 10 bis 50 bar. Das molare Verhältnis von Edukt zu Wasserstoff wird im Bereich von etwa 1 : 100 bis etwa 1 : 2000, wie z.B. 1 : 800 bis 1 : 1500 eingestellt.

Für die erfindungsgemäße Hydrierreaktion brauchbare Katalysatoren sind dem Fachmann bekannt. So können beispielsweise verschiedene Kupferkatalysatoren eingesetzt werden. Im Stand der Technik wird beispielsweise die Verwendung reduzierter Kupferchromit-Katalysatoren beschrieben, welche unter der Bezeichnung 85/1 bei Davy Process Technology Ltd., England, erhältlich sind. Erfindungsgemäß besonders geeignet sind jedoch geträgerte Kupfeoxidkatalysatoren, wobei das Kupferoxid auf Aluminiumoxid- oder Siliciumdioxid-Trägermaterialien aufgebracht ist. Beispiele zur Hydrierung von Bernsteinsäure-Ester zu BDO/GBL/THF mit Kupfer-Katalysatoren sind auch in der folgenden Dissertation beschrieben: Schlander, Jan, Feb. 2000, Universität Karlsruhe, "Gasphasenhydrierung von Maleinsäuredimethylester zu 1,4-Butandiol, gamma-Butyrolacton und Tetrahydrofuran an Kupfer-Katalysatoren".

### e) Fermentative Herstellung von Carbonsäure-Ammoniumsalzen

Die fermentative Herstellung wässriger Ammoniumsalzlösungen von Polycarbonsäuren ist an sich aus dem Stand der Technik bekannt. So beschreibt beispielsweise das US-Patent 5,869,301 die fermentative Herstellung von Carbonsäuren, wie insbesondere Succinat, aus Glucose unter Verwendung von verschiedener Mikroorganismen, insbesondere Bakterien der Gattung Escherichia.

Als Beispiele für geeignete natürliche oder rekombinante, pro- oder eukaryotische Mikroorganismen sind solche zu nennen, die unter aeroben oder anaeroben Bedingungen zur fermentativen Produktion der gewünschten Polycarbonsäure geeignet sind, Insbesondere sind Bakterien zu nennen, wie solche der Gattung Escherichia, Bacteroides, Anaerobiospirillum,Actinobacillus und Mannheimia und Pilze der Gattung Rhizopus und Aspergillus.

Geeignete Fermentationsbedingungen, Medien, Fermenter und dergleichen sind vom Fachmann im Rahmen seines allgemeinen Fachwissens festlegbar. Dazu kann er sich z.B. der Ausführungen in geeigneter Fachliteratur, wie z.B. Rehm et al, Biotechnology, Vol. 3 Bioprocessing, 2nd Ed., (Verlag Chemie, Weinheim), bedienen.

Dazu wird ein steriles Kulturmedium hergestellt, das das Substrat sowie weitere die zum Wachstum des Mirooganismus und der Produktbildung gegebenenfalls erforderliche Zusätze, wie Kohlenstoff- und/oder Stickstoffquellen, Spurenelemente und dergleichen enthält, und mit einer geeigneten Menge einer fFrischen Vorkultur des Mikroorganismus angeimpft wird. Die Kultivierung erfolgt in einem geeigneten Fermenter kontinuierlich oder diskontinuierlich wobei sich die Polycarbonsäure (bzw. Salze davon) im Medium anreichert. Während der Fermentation wird der pH-Wert des Fermentationsmediums durch Zugabe von Ammoniumhaltigen-Lösungen, wie beispielsweise Ammoniumhydroxid oder Ammoniumcarbonat eingestellt um eine Inhibierung der Kultur zu vermeiden. Prinzipiell ist aber auch die Einleitung von Ammoniak oder die Verwendung einer Ammoniumhydrogencarbonatlösung denkbar. Nach beendeter Fermentation kann die Fermentationsbrühe, welche das Ammoniumsalz der Polycarbonsäure, vorzugsweise gelöst, enthält, entweder direkt der erfindungsgemäßen Veresterungsreaktion zugeführt werden. Vorzugsweise wird jedoch zunächst Biomasse, beispielsweise durch Zentrifugation oder Filtration, abgetrennt. Gegebenenfalls nach dem Waschen der abgetrennten Biomasse und Vereinigung der Waschflüssigkeit mit der Ammoniumsalzlösung kann die Salzkonzentration, falls zu niedrig, beispielsweise durch destillative Abtrennung von Wasser, weiter erhöht werden, so dass eine konzentrierte wässrige Ammoniumsalz-haltige Phase gebildet wird, die einen prozentualen Gehalt an Salz der Polycarbonsäure im Bereich von etwa 10 bis 70, vorzugsweise 30 bis 50 Gew.-% aufweist. Dieses wässrige Konzentrat wird dann der Veresterungsreaktion zugeführt.

In einer Ausführungsform des erfindungsgemäßen Verfahrens wird die Fermentationsbrühe hergestellt durch anaerobe oder aerobe Fermentation oder einer Kombination aus aerober und anaerober Fermentation, bevorzugt durch die Fermentation von Kohlenstoffquellen, wie beispielsweise CO₂, Ölen oder Alkoholen, insbesondere Glycerin, Ethanol, Methanol, oder Sorbit, oder Zuckern, wie z.B. C₆- oder C₅-Zuckern, insbesondere Glucose, Sorbose, Arabinose, Xylose oder Saccharose, in Reinform oder z.B. als Melasse oder Mischungen der genannten Stoffe oder deren Vorprodukten, wie z. B. Stärke, beispielsweise zusammen mit Enzymen, oder anderen möglichen kohlenstoffhaltigen Zusammensetzungen, wie z. B. Cellulose, z.B. in Form von Altpapier, Holzabfällen oder Bestandteilen von stärkehaltigen Pflanzen.

Zur Fermentation werden beispielsweise prokaryotische oder eukaryotische Mikroorganismen verwendet, beispielsweise Bakterien, wie E. coli, Anaerobiopirillum succiniproducens, Actinobacillus succinogenes, Mannheimia succiniproducens oder andere Bakterien oder Pilze, die die gewünschte Carbonsäure, z.B. Bernsteinsäure, produzieren.

In einer Ausführungsform wird in der Fermentation ein Mikroorganismus, beispielsweise E.coli, nach einer aeroben Wachstumsphase zur Entwicklung von Biomasse zu anaeroben Bedingungen transferiert. In dieser anaeroben Phase findet die Synthese von Succinat statt. Beide Kultivierungsschritte können insbesondere in Komplexmedium stattfinden. In einer weiteren Ausführungsform erfolgt in der Fermentation die Kultivierung des Mikroorganismus, insbesondere von E. coli, in der anaeroben Phase mit Minimalmedium. Eine weitere Ausführungsform umfasst das mehrmalige Recycling der Zellen und Durchführung der anaeroben Produktionsphase auf Komplex- oder Minimalmedium.

Die Fermentation kann in Rührfermentern, Blasensäulen und Schlaufenreaktoren durchgeführt werden. Ein ausführlicher Überblick über die möglichen Ausführungsarten inkl. Rührerformen und geometrischer Ausführungen ist in "Chmiel: Bioprozesstechnik: Einführung in die Bioverfahrenstechnik, Band 1" zu finden. Bei der Prozessführung stehen typischerweise die folgenden, dem Fachmann bekannten oder z. B. in "Chmiel, Hammes und Bailey: Biochemical Engineering" erläuterten Varianten, wie Batch, Fed-Batch, Repeated Fed-Batch oder aber auch kontinuierliche Fermentation mit und ohne Rückführung der Biomasse zur Verfügung. Je nach Produktionsstamm kann/muss eine Begasung mit Luft, Sauerstoff, Kohlendioxid, Wasserstoff, Stickstoff oder entsprechenden Gasmischungen erfolgen, um gute Ausbeuten zu erzielen.

Vor der Umsetzung in der Fermentationsbrühe in dem erfindungsgemäßen Verfahren kann die Fermentationsbrühe vorbehandelt werden, beispielsweise kann die Biomasse der Brühe abgetrennt werden. Verfahren zur Abtrennung der Biomasse sind dem Fachmann bekannt, wie z.B. Filtration, Sedimentation und Flotation. Folglich kann die Biomasse beispielsweise mit Zentrifugen, Separatoren, Dekantern, Filtern oder in Flotationsapparaturen abgetrennt werden. Zur möglichst vollständigen Gewinnung des Wertproduktes empfiehlt sich oftmals ein Waschen der Biomasse, z. B. in Form einer Diafiltration. Die Wahl der Methode ist abhängig von dem Biomassenanteil in der Fermenterbrühe und den Eigenschaften der Biomasse, sowie der Interaktion der Biomasse mit dem Wertprodukt. Die Fermentationsbrühe kann in einer Ausführungsform sterilisiert oder pasteurisiert werden.

In einer weiteren Ausführungsform wird die Fermentationsbrühe aufkonzentriert. Diese Aufkonzentrierung bzw. Eindampfung kann je nach Anforderung diskontinuierlich, oder kontinuierlich geschehen. Dabei sollten Druck- und Temperaturbereich so gewählt werden, dass zum einen keine Produktschädigung auftritt, zum anderen ein minimierter Einsatz von Apparaten und Energie notwendig ist. Insbesondere die geschickte Wahl der Druck- und Temperaturstufen für eine mehrstufige Verdampfung ermöglicht eine Einsparung von Energie.

Apparativ können dazu Rührkessel, Fallfilm-, Dünnschicht, Zwangsentspannungsumlauf-, und sonstige Verdampferbauarten in Natur- oder Zwangsumlauffahrweise genutzt werden.

Folglich wird unter dem Begriff "Fermentationsbrühe" eine auf einen fermentativen Prozess beruhende wässrige Lösung verstanden, die nicht oder beispielsweise wie hierin beschrieben aufgearbeitet wurde.

### Experimenteller Teil

Allgemeine Angaben:
Analyse der Säuren/Ester/Hydrierungsprodukte durch GC;
Verwendete Säule: CP-SIL 19CB, 25 m, 0,32 mm, 1,2µm;
Injektor: 235°C;
FID: 300°C

### Beispiel 1: Herstellung von Bernsteinsäuredibutylester mittels Reaktivdestillation in einer Glockenbodenkolonne (Einspeisung von dampfförmigem Butanol in den Kolonnenboden)

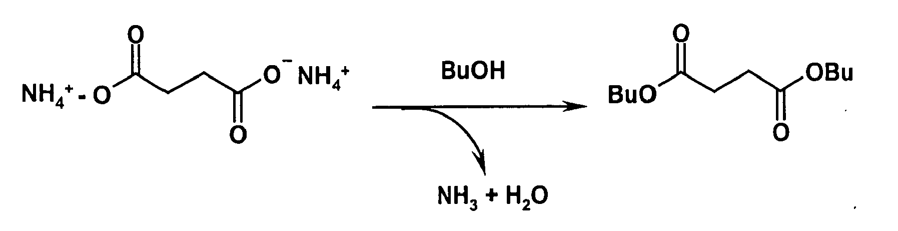

Für die Veresterungsreaktion wird eine Glockenbodenkolonne (durch evakuierten Doppelmantel isoliert) (Durchmesser: 0,054 m, Anzahl der Böden:10) verwendet, deren Sumpf mit einem beheizten Rührbehälter (Größenangabe: 2 Liter) verbunden ist, in welchem n-Butanol vorgelegt ist und für die Umsetzung unter Rühren zum Sieden gebracht wird. Der Kolonnenkopf ist über eine Zuführung und eine HPLC-Pumpe mit einem Vorlagenbehälter verbunden. Am Kolonnenkopf befindet sich weiterhin eine mit einem Kondensator und einem Phasentrenngefäß verbundene Ableitung für die während der Reaktivdestillation abdestillierbaren Komponenten sowie ein Rücklauf für zurückgewonnenes flüssiges Kondensat.

Zur Durchführung der Reaktivdestillation wird aus dem Rührbehälter, in dem zu Beginn reines n-Butanol (1 Liter) vorgelegt wird, Butanoldampf in die Kolonne eingeleitet und die Kolonne im totalen Rücklauf betrieben. Sobald das Temperaturprofil (Siedetemperatur BuOH in der Kolonne) in der Kolonne stationär ist, wird eine wässrige Lösung von 558,86 g Bernsteinsäurediammoniumsalz (Diammoniumsuccinat (DAS), DAS-Konzentration = 24%), versetzt mit 3,3 g para-Toluolsulfonsäure, mittels der HPLC-Pumpe, bei Normaldruck und am Kopf der Glockenbodenkolonne bei einer Temperatur zwischen 90 (am Kolonnen-Kopf) bis 118 °C (im Sumpf, zu Beginn reines Butanol) und einer Zulaufgeschwindigkeit von 0,2 - 0,7 ml/min zudosiert.

Während der Reaktion wird im Rührbehälter der gebildete Bernsteinsäuredibutylester (BDBE: Sdp = 278°C bei Normaldruck) im Sumpf angereichert (zusammen mit Butanol). Die Innentemperatur im Rührbehälter steigt während der Synthese von 118,3 °C auf 128,8° C. Über Kopf wird das Azeotrop Wasser / n- Butanol und NH₃ abdestilliert und zum Teil kondensiert. Die nicht kondensierten Bestandteile (vermutlich vor allem NH₃) können die Apparatur in einem Abzweig als Abluft verlassen. In einem Phasentrenngefäß wird das Kondensat in eine Butanol- und eine Wasserphase getrennt. Die Butanol-Phase wird am Kopf in die Kolonne über den Kondensatorrücklauf zurückgeführt.

### Versuchsergebnis:

DAS eingesetzt: 558,86 g
p-Toluolsulfonsäure: 3,3 g
Ausbeute (nach GC-Analyse):
   Bernsteinsäuredibutylester 96,5 %,
   Bernsteinsäureanhydrid 0,3 %,
   Amido-Butylester 0,1
   Succinimid <<0,1 %
Gesamt-Stickstoffgehalt 120 ppm.

Der Vesterungsaustrag wurde filtriert (Bernsteinsäure und pTsOH fallen aus) und kann dann ohne weitere destillative Reinigung direkt weiterhydriert werden.

### Beispiel 2: Katalytische Hydrierung von Bernsteinsäuredibutylester zu THF

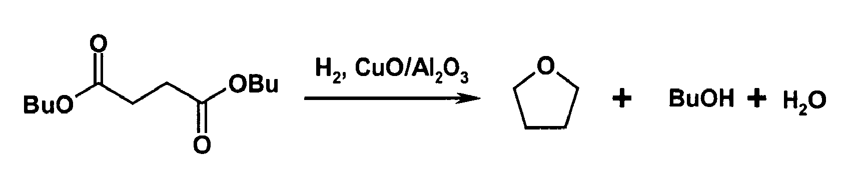

Für die kontinuierliche (in der Gasphase) Hydrierung besteht die Druckapparatur aus einem Verdampfer (T= 250°C), einem Reaktor, einem Kühler, einer Wasserstoffzufuhr, einer Abgasleitung und einem Kompressor. Der Druck in der Apparatur wird bei 13 bar konstant gehalten. Der Bernsteinsäuredibutylester wird verdampft und mit frischem Wasserstoff und Kreisgas (mol(BSDBE):mol(H₂)=1:1000) gemischt. Die Mischung aus Wasserstoff und Ester gelangt in einen Reaktor (240°C) mit Katalysator (120ml CuO/Al₂O₃) gefüllt. Die Produkte werdenim Kühler kondensiert.

Versuchsbedingungen: T(Verdampfer)=250°C, T(Reaktor)=240°C, Druck= 13bar, Belastung= 0,1 kg_{Ester}/I_{Kat}.h, Mol-Verhältnis Edukt:H₂=1:1000

Ausbeute (nach GC-Analyse; Vollumsatz von Bernsteinsäuredibutylester):
Dibutylester eingesetzt: 2825g
   THF= 69,0 %,
   BDO= 0,0 %,
   GBL= 0,0%
Andere Nebenprodukte sind in geringen Mengen Butanol und Dibutylether; Verlust von THF wegen der Strippung (N₂).
Gesamt-Stickstoffgehalt des Produkts: 110 ppm

### Referenzbeispiel 1: Versuche zur direkten Katalytischen Hydrierung von Bernsteinsäuresalzen zu THF

Zu Vergleichszwecken wurden verschiedene Salze der Bernsteinsäure einer homogenen bzw. heterogenen Hydrierung unterzogen. Man beobachtet entweder keine Reaktion (Dinatriumsalz) oder die Bildung verschiedener Hydrierungsprodukte, wie BDO, GBL oder Pyrrolidon, nicht jedoch die Bildung des gewünschten Produkts THF

| | | Produktausbeuten | | | |
|---|---|---|---|---|---|
| Substrat | Katalysator^{(a)} | 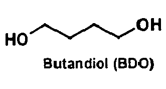 | 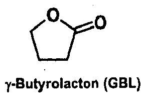 | 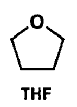 | 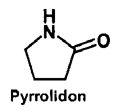 |
| 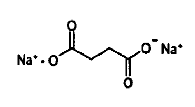 | Ru/Triphos | keine Reaktion | | | |
| | Sn/Pt | keine Reaktion | | | |
| 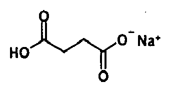 | Ru/Triphos | 21,3% ^{(b)} | 3,3% ^{(b)} | - | - |
| | Sn/Pt | 10%^{(b)} | 26,2% ^{(b)} | - | - |
| 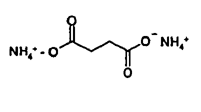 | Ru/Triphos | 8,6% | 3,7% | - | 4,2% |
| | Sn/Pt | - | 2,5% | - | 4,8% |

| | | | | | |
|---|---|---|---|---|---|
| ^{(a)} Homogene Hydrierungsbedingungen: Ru(acac)₃/Triphos, 150°C, 150 bar H₂, 24h. Heterogene Hydrierungsbedingungen: 3% Sn /7% Pt on C, 120°C, 200 bar H₂, 24h. ^{(b)} Das Mononatriumsalz kann als ein 50:50 Gemisch aus Disäure und Dinatriumsalz angesehen werden. Nur die Disäure konnte in Butandiol BDO and GBL. Es wurde keine Konversion eines Natriumcarboxylats beobachtet. | | | | | |

### Referenzbeispiel 2: Vorversuche zur direkten Veresterung verschiedener Dicarbonsäuresalze mit Butanol

Verschiedene Alkali- und Erdalkali-Salze von Bernsteinsäure sowie das Diammoniumsalz von Fumarsäure wurden in einem Rührkolben mit Butanol verestert (10g Salz + 1g pTsOH in BuOH, Rückfluss 96h) Die Versuchsergebnisse sind in folgender Tabelle zusammengefasst.

Außerdem ist das erfindungsgemäße Ergebnis der Umsetzung von Diammoniumbernstainsäure zum Vergleich nochmals aufgeführt.

Man beobachtet überraschenderweise, dass von den zusätzlich getesteten Bernsteinsäuresalzen keines in den gewünschten Diester überführt wird. Das Ammoniumsalz der Fumarsäurekonnte mit geringerer Ausbeute also das entsprechende Bernsteinsäuresalz verestert werden.

| | | Produktausbeute | | | |
|---|---|---|---|---|---|
| Substrat | Katalysator ^{(a)} | 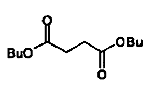 | 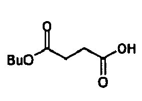 | 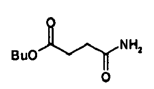 | Andere Nebenprodukte ^{(b)} |
| 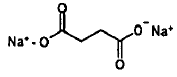 | 0,5% pTsOH | Keine Reaktion | | | |
| 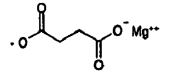 | 0,5% pTsOH | Keine Reaktion | | | |
| 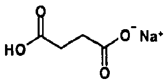 | 0,5% pTsOH | Spuren ^{(c)} | Spuren ^{(c)} | - | - |
| 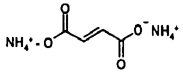 | 10% pTsOH | 76% ^{(d)} | - | - | - |
| 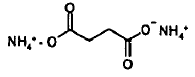 | 0,5% pTsOH | 97% | - | 140 ppm ^{(e)} | - |

| | | | | | |
|---|---|---|---|---|---|
| ^{(a)} p-TsOH: para-Toluolsulfonsäure ^{(b)} Alle im GC beobachteten Produkte wurden durch GC-MS Analyse identifiziert. ^{(c)} Das Salz war unlöslich in Butanol, daher konnte nur die Mono- und Dibutylester haltige Lösung im GC analysiert werden. Das Mononatriumsalz kann als ein 50:50 Gemisch aus Disäure und Dinatriumsalz angesehen werden. Nur die Disäure ist veresterbar. Es wurde keine Umsetzung eines Natriumcarboxylats nachgewiesen. ^{(d)} Ziel dieses Experiments war es, zu prüfen, ob das Fumarsäuresalz in den Dibutylester überführbar ist. Es wurde ein unlöslicher Feststoff beobachtet (unumgesetztes Fumarsäuresalz). ^{(e)} Elementaranalyse | | | | | |

### Bezugszeichenliste

- 1: Vorlagenbehälter (Ammoniumsalz-Lösung)
- 2: Rührbehälter (Alkohol-Vorlage, Verdampfer, Sumpf)
- 3: Glockenbodenkolonne
- 4: Glockenboden
- 5: unteres Ende der Kolonne
- 6: Kolonnen-Kopf
- 7: Zuleitung Vorlage
- 8: Verbindung Kolonne-Rührbehälter
- 9: Rührer
- 10: Ableitung Azeotrop (Brüdenleitung)
- 11: Kondensator
- 12: Ableitung
- 13: Phasentrenngefäß
- 14: Abgasleitung
- 15: Ableitung Wasserphase
- 16: Rückführung organische Phase

- 101: Vorlagenbehälter (Ammoniumsalz-Lösung)
- 102: Vorlagenbehälter Alkohol
- 103: Zuleitung Ammoniumsalz-Lösung (aus Vorstufe)
- 104: Zuleitung Alkohol
- 105: Zuleitung Ammoniumsalzlösung
- 106: Zuleitung Alkohol
- 107: Kolonne
- 108: Verdampfer
- 109: Abzug von Rückständen (optional)
- 110a: Sumpf-Abzug: Ester-(Alkohol)-Gemisch
- 110b: Rückführung Sumpf
- 110c: Zuleitung Kolonne
- 111a: Rückführung organische Phase
- 111 b: Rückführung wässrige Phase (optional)
- 112: Phasentrenngefäß
- 113: Kondensator
- 114: Abgasleitung
- 114a: Ableitung
- 115: Ableitung Wasserphase
- 116: Ableitung Azeotrop (Brüdenleitung)
- 117: Kolonnen-Kopf
- 118: Sumpf
- 119: Einbauten (wie Glockenboden)

## Patentansprüche

1. Verfahren zur Herstellung eines Polycarbonsäurealkylesters, wobei man eine Reaktivdestillation durchführt, indem man eine Vorlage, umfassend eine wässrige Lösung eines Ammoniumsalzes der Polycarbonsäure in einer Destillationskolonne mit Alkanol in Kontakt bringt und bei der Veresterung anfallenden Ammoniak und Wasser destillativ aus der Reaktion entfernt.

2. Verfahren nach Anspruch 1, wobei sich der gebildete Carbonsäureester im Sumpf der Kolonne anreichert.

3. Verfahren nach Anspruch 1 oder 2, wobei man das Salz einer Carbonsäure einsetzt, die ausgewählt ist unter jeweils gesättigten oder ungesättigten C₃ - C₂₀-Polycarbonsäuren.

4. Verfahren nach einem der vorhergehenden Ansprüche, wobei man ein Alkanol einsetzt, das ausgewählt ist unter geradkettigen oder verzweigten C₁- C₆-Monoalkanolen.

5. Verfahren nach einem der vorhergehenden Ansprüche, wobei die Carbonsäure mit Ammoniak teilweise oder vollständig neutralisiert ist.

6. Verfahren nach einem der vorhergehenden Ansprüche, wobei man die Reaktion in Gegenwart eines Veresterungskatalysators durchführt,

7. Verfahren nach einem der vorhergehenden Ansprüche, wobei man die wässrige Carbonsäuresalz-haltige Lösung in der oberen Hälfte der Destillationskolonne zuführt.

8. Verfahren nach einem der vorhergehenden Ansprüche, wobei man freigesetzten Ammoniak und Wasser über den Kolonnenkopf abdestilliert.

9. Verfahren nach Anspruch 8, wobei man ein Alkanol/Wasser-Azeotrop und Ammoniak abdestilliert, kondensiert, aus dem Kondensat das Alkanol gewinnt und in die Umsetzung zurückführt.

10. Verfahren nach Anspruch 8, wobei man ein Alkanol/Wasser-Hetero-Azeotrop und Ammoniak abdestilliert, kondensiert, aus dem Kondensat die organische Alkanol-Phase abtrennt und in die Umsetzung zurückführt.

11. Verfahren nach einem der vorhergehenden Ansprüche, wobei man einen homogenen oder einen heterogenen Katalysator verwendet.

12. Verfahren nach Anspruch 11, wobei der heterogene Katalysator ein saurer lonenaustauscher-Katalysator ist.

13. Verfahren nach Anspruch 11 oder 12, wobei der heterogene Katalysator auf oder in Kolonneneinbauten fixiert ist.

14. Verfahren nach Anspruch 11, wobei man einen homogenen Katalysator verwendet, den man der wässrigen Ammoniumsalz-Lösung der Carbonsäure zusetzt.

15. Verfahren nach Anspruch 14, wobei der eingesetzte homogene Katalysator p-Toluolsulfonsäure ist.

16. Verfahren nach einem der vorhergehenden Ansprüche, wobei das eingesetzte Carbonsäure-Ammoniumsalz Diammoniumsuccinat ist.

17. Verfahren nach einem der vorhergehenden Ansprüche, wobei das eingesetzte Carbonsäure-Ammoniumsalz chemisch hergestellt ist.

18. Verfahren nach einem der Ansprüche 1 bis16, wobei die Vorlage eine gegebenenfalls vorgereinigte und/oder aufkonzentrierte Fermentationsbrühe umfasst.

19. Verfahren nach Anspruch 18, wobei die Fermentationsbrühe aus der Kultivierung eines Pentosen und/oder Hexosen, insbesondere Glucose-fermentierenden Mikroorganismus stammt

20. Verfahren nach einem der vorhergehenden Ansprüche, wobei das eingesetzte Alkanol n-Butanol ist.

21. Verfahren nach Anspruch 20, wobei man Di-n-butyl-succinat erhält.

22. Verfahren zur Hydrierung von Polycarbonsäurealkylestern, wobei man zunächst eine Veresterungsreaktion nach einem der vorhergehenden Ansprüche durchführt, aus dem dabei anfallenden Ester-haltigen Reaktionsgemisch den Carbonsäurealkylester gegebenenfalls aufreinigt, den Ester katalytisch hydriert und das gewünschte Wertprodukt isoliert.

23. Verfahren nach Anspruch 22, wobei man einen geträgerten CuO-Katalysator für die Hydrierung verwendet.

24. Verfahren nach Anspruch 23, wobei man Di-n-butyl-succinat zu Tetrahydrofuran hydriert.

25. Verfahren nach einem der Ansprüche 22 bis 24, wobei man Di-n-butyl-succinat zu 1,4-Butandiol hydriert.

26. Verfahren nach einem der Ansprüche 22 bis 24, wobei man Di-n-butyl-succinat zu gamma-Butyrolacton hydriert.

27. Verfahren nach einem der Ansprüche 22 bis 24, wobei man Di-n-butyl-succinat zu einer Mischung aus THF und/oder 1,4-Butandiol und/oder gamma-Butyrolacton hydriert.
